Europäisches Patentamt

European Patent Office

Office européen des brevets

Publication number: **0 280 414**

**A1**

## EUROPEAN PATENT APPLICATION

Application number: **88300789.0**

Date of filing: **29.01.88**

Int. Cl.⁴: **A61F 9/00 , G02B 27/00**

Priority: **02.02.87 US 9724**

Date of publication of application:
**31.08.88 Bulletin 88/35**

Designated Contracting States:
**AT BE CH DE ES FR GB GR IT LI LU NL SE**

Applicant: **TAUNTON TECHNOLOGIES, INC.**
**631 Main Street**
**Monroe Connecticut 06468(US)**

Inventor: **Telfair, William B.**
**24 Pocono Road**
**Newtown Connecticut(US)**
Inventor: **Yoder, Paul R., Jr.**
**9 Bhasking Ridge Road**
**Wilton Connecticut(US)**
Inventor: **Martin, Clifford A.**
**64 Beachview Avenue**
**Bridgeport Connecticut(US)**

Representative: **Milhench, Howard Leslie et al**
**R.G.C. Jenkins & Co. 26 Caxton Street**
**London SW1H 0RJ(GB)**

Sculpture apparatus for correcting curvature of the cornea.

In the context of ultraviolet-laser sculpting of the cornea to achieve optical correction through a newly shaped anterior surface, the invention subjects the laser beam to certain shaping and homogenizing operations prior to any attempt to specially characterize the beam for a particular sculpture procedure. In a preferred embodiment, an excimer laser (11) produces a beam of rectangular section, and the short dimension of this section is optically expanded (50, 51) to produce a beam section having quadrature profiles of substantially equal extent on opposite sides of the central axis of the expanded beam; the expanded beam is thereafter subjected to optical rotation (52) in the course of a given surgical exposure to the cornea, whereby irregularity in the intensity profile of the expanded beam is converted to an irregularity of rotational symmetry. A compensating filter (55) having an attenuating profile of rotational symmetry effectively offsets irregularity of circular symmetry in the time-integrated product of beam rotation.

FIG. 2.

## SCULPTURE APPARATUS FOR CORRECTING CURVATURE OF THE CORNEA

### RELATED CASE

This application is directed to certain refinements of and improvements over the disclosure of our pending European Patent Application No. 87310283.4, filed November 20, 1987.

### BACKGROUND OF THE INVENTION

The invention relates to that aspect of opthalmic surgery which is concerned with laser operations upon the external surface of the cornea, such operations involving controlled ablation of the cornea with penetration into the stroma and volumetric removal of corneal tissue whereby said external surface is characterized by a sculptured new curvature having improved optical properties.

Several different techniques and related apparatus are described for such sculpture of the cornea in pending L'Experance European patent applications nos. EP-A-0209992, 86304097, EP-A-0218427 and 87306826 to which reference may be made for greater understanding. Suffice it to say that these techniques rely on ultraviolet radiation which is preferably of less than 200-nm wavelength, as is provided by an excimer laser operating with argon fluoride. Typical beam dimensions of the excimer laser are rectangular, and EP-A-0218427 discloses a circular opening in a mask for reducing the laser beam to a cylinder of circular section; thereafter, the cylindrical beam is variously characterized so that at incidence with the cornea and on the eye axis, the distribution of laser-flux density will be a correspondingly distributed pattern of cornea-curvature correction.

In European patent application no. 87306826, the sculpturing result for cornea-curvature correction is achieved by exposing the cornea to a sequence of mask openings, of different but related areas, whereby the cumulative effect is to so expose certain areas more in relation to others that the consequence is the desired net curvature change.

The techniques of both EP-A-0218427 and European patent application no. 87306826 involve non-scanning use of the involved laser beam, and they assume that a sufficiently homogenous beam will be available prior to characterizing the same for sculpturing delivery to the cornea. But it has been found that the flux-density distribution within such a beam is not necessarily uniform and that it can vary with time, thus presenting the possibility of impaired quality of the intended curvature correction. Said relating European patent application no. 87310283.4 and the presnt invention both address this specific problem.

### BRIEF STATEMENT OF THE INVENTION

It is an object of the invention to provide a method and means for improving the quality of laser-sculpted curvature correction of the cornea.

It is a specific object to achieve the above object by improving the homogeneity of laser-flux distribution prior to characterizing the distribution for specific different curvature-correction purposes.

It is also a specific object to improve the quality of characterized laser radiation delivered for corneal surgery.

Another specific object is to achieve the above objects with means for monitoring flux distribution to determine its acceptability vel non for the intended surgical purpose.

A still further object is to provide means for automated termination of laser-flux delivery to an eye, in the event that predetermined criteria of laser-flux distribution are not tolerably met.

In its broadest aspect, the present invention provides laser apparatus for ophthalmological surgery including beam rotation means provided in the laser beam path for alleviating the effects of irregular beam intensity distribution across the beam cross-section.

As is described particularly hereinafter, the invention achieves the foregoing objects by subjecting a laser beam to certain shaping and homogenizing operations prior to any attempt to specially characterize the beam for a particular sculpturing surgical procedure. In a preferred embodiment, beam-rotation about the axis of the laser beam is an important manipulative step (following optical expansion of the short dimension of the cross-section of the laser beam), whereby to achieve homogeneity in the sectional distribution of radiation intensity, as integrated for tissue-ablating purposes in the course of a predetermined period of surgical exposure

### DETAILED DESCRIPTION OF THE INVENTION

The invention will be illustratively described for preferred and other embodiments, in conjunction with the accompanying drawings, in which:

Fig. 1 is a simplified block diagram to show the functional relationship of generalized optical, mechanical and electrical components of apparatus incorporating the invention;

Fig. 2 is an expanded schematic diagram of some of the optical components of Fig. 1;

Fig. 2a is a fragmentary diagram to show an optical feature for certain components of Fig. 2;

Figs. 3a, 3b, and 3c respectively depict beam-section area, and horizontal and vertical beam-intensity profiles for a first condition in the apparatus of Fig. 1;

Figs. 4a, 4b, 4c and 5a, 5b, and 5c correspond to Figs. 3a, 3b, and 3c for second and third conditions;

Fig. 6 is a graph of an optical-filter characteristic;

Figs. 7a, 7b, and 7c correspond to the Fig. 3, 4, and 5 groupings, to show a fourth condition;

Figs. 8a, 8b, and 8c correspond to the Fig. 7a, 7b, and 7c grouping, to illustrate an irregularity condition as to which Fig. 8d applies to another irregularity which differs from that of Fig. 8c;

Figs. 9a, 9b, and 9c correspond to Figs. 8a, 8b, and 8c to depict a further condition, together with graphical showing at Figs. 9d, 9e of means for correcting this further condition;

Figs. 10a, 10b, 10c are schematic diagrams to illustrate modification of a portion of the optical system of Fig. 2; and

Fig. 11 is an isometric diagram in illustration of beam rotation in use of the invention.

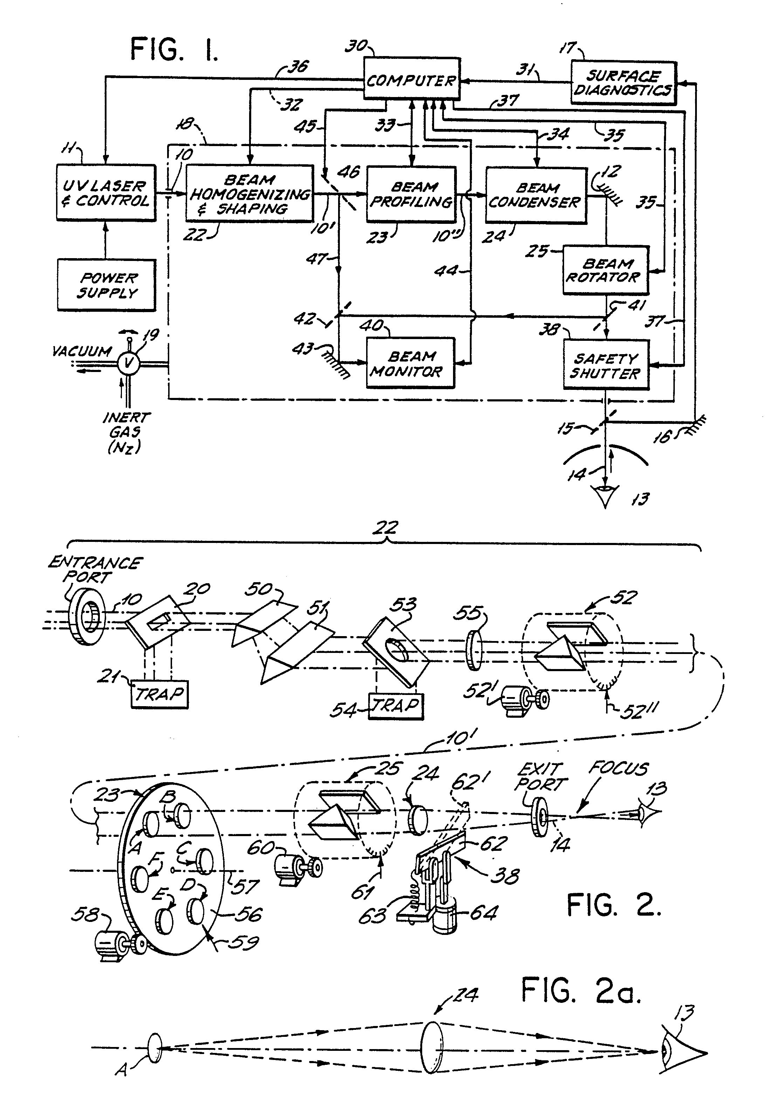

In Fig. 1, the invention is shown in conjunction with apparatus for delivery of the beam output 10 of an ultraviolet laser 11 along an optical path which is horizontal until folded at 12 for vertically downward fixed-axis passage to the eye 13 of a patient, it being understood that the patient may be suitably restrained, facing up, with the eye 13 also retained for coincidence of the visual axis with the axis 14 of impinging laser radiation. In preparation for laser surgery upon the anterior surface of eye 13, illuminating and reflecting components of a corneascope or other means of evaluating anterior topography of eye 13 will have been indexed into alignment with axis 14, as by mounting such components on an index arm which can be selectively swung into and out of topography-measuring position; in the drawing, these illuminating and reflecting components are collectively symbolized by folding mirrors 15-16, and camera and display components of the corneascope will be understood to be part of surface-diagnostic means 17.

The invention is primarily concerned with means for processing and monitoring the output beam 10, for assuring the safety and quality of the radiation delivered at 14 for operation on eye 13. And the optical means for such processing and monitoring are preferably contained within a sealed enclosure 18, whereby a suitably inert gas environment may assure against such beam-degradation as would occur from ozone development in an air environment; legends applied to the respective inlets of valve 19 suggest that the inert environment may be provided by a supply of dry nitrogen gas, following evacuation of air from enclosure 18.

One of the existing commercial ultraviolet-laser products of Questek Inc., Billerica, Massachusetts, for example their Model 2460 excimer laser operating with argon-fluoride, is satisfactory for use as laser 11. For this product, energy per pulse is selectively variable up to 275 millijoules, pulse width is in the range 8 to 20 nanoseconds, and pulse-repetition rate is selectively available up to 150 Hz, being typically and preferably in the range 5 to 15 Hz for presently described purposes; full rated power is not necessarily required by the invention, but this laser includes its own built-in microprocessor to control laser output power, gas filling and laser-systems diagnostics, whereby predetermined output power can be automatically maintained for an extended useful life, as compared with other excimer lasers.

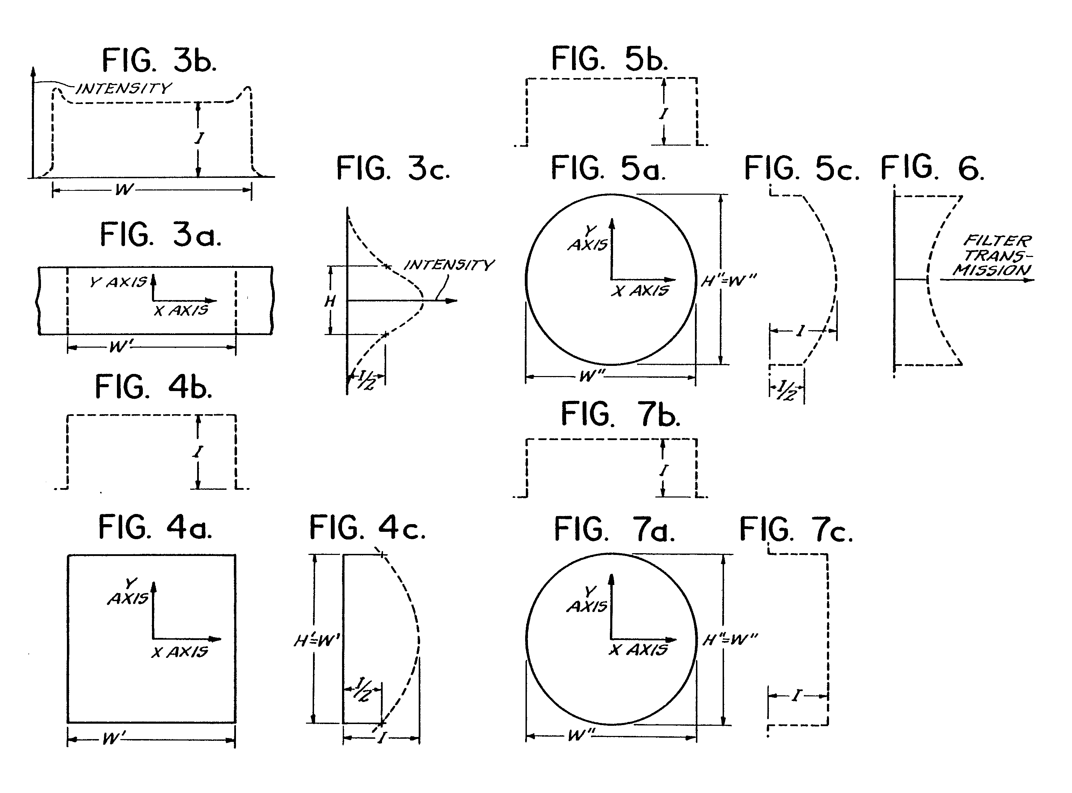

Laser 11 emits a collimated beam 10 of typical approximate sectional dimensions 22-mm by 7-mm, wherein the long dimension is horizontal and will be referred to as the width dimension (W) along the X axis, and the short dimension is vertical and will be referred to as the height dimension (H) along the Y axis. These dimensions are stated as approximate because, as indicated for the spatial (section) and width distribution views of Figs. 3a and 3b, the generally rectangular emitted-beam section is characterized by laterally extending irregular fringes of greater relative intensity at both ends of the width dimension. These fringes are picked off by a scraper 20 (Fig. 2) for dissipation at a trap 21; scraper 20 has an elongate rectangular opening in its reflecting surface, whereby the scraped beam is characterized by the slightly reduced width W' for a more regular rectangular section area wherein the width (W') still greatly exceeds the height dimension H. As best seen in Figs. 3b and 3c, the cross-sectional intensity profiles are different in the X and Y directions. In the X direction, the profile is essentially a "flat top", in that the higher end peaks have been discarded by the scraper; and in the Y direction, the intensity distribution is substantially Gaussian about the midpoint of the H dimension. For the dimensional legends applied to Fig. 3c, the scraper 20 will be seen to have selected that part of the Y-axis intensity distribution which exceeds half the maximum.

The scraper 20 is but one of a succession of optical elements of first beam-processing means 22, generally indicated in Fig. 1 as beam homogenizing and shaping means, the function of which is to preset at path location 10' an acceptably homogeneous collimated beam of circular section, wherein the circle diameter is considerably larger (e.g., 14-mm) than the maximum 5 to 7-mm diameter characterized beam ultimately delivered at 14 to the eye.

The large homogeneous circular beam 10' is then subjected to further processing at 23, for profiling purposes. This may involve applying characterized reflectance or filtering treatment to the beam, in accordance with teachings in EP-A-0218427, or it may involve applying a characterized succession of mask openings to control a particular succession of lapped areas of beam projection in the course of a given surgical procedure, in accordance with teachings in European patent application no.87306826. After this second or profiling operation at 23, the beam at location 10" is fully characterized (whether by area distribution of intensity (I) or by time distribution of correlated lapping areas), but to a scale which is preferably in the order of at least twice the scale desired for eye delivery at 14. Third beam-processing means 24 which is labeled "beam condenser", but which will be understood to include a single lens or a multiplicity of lenses having positive net optical power, a fixed-focus telescope or a zoom telescope, should scale adjustability be desired, brings the characterized beam to the desired scale for surgical delivery at 14.

For those situations in which an astigmatic error is to be correctively improved, the characterization at 23 will be understood to be such as to develop characterizing symmetry on laterally opposite sides of a single diametric axis across the circle of the laser beam, and a beam rotator 25 is provided on the optical path prior to delivery via beam 14, to enable the surgeon to set the orientation axis of astigmatic-error correction, based on the presurgical diagnosis of the particular eye 13; in an alternate configuration, the characterization means 23 may be independently rotatable about the laser-beam axis, through action of an associated rotary-drive motor in conjunction with angular-position-sensing means (not shown).

A beam monitor 40 is shown to be supplied, via beam-splitter pick-off at 41 at folding mirrors 42, 43, with a fraction of the total beam that has been characterized and scaled down for surgical delivery. This fraction is for the total section of the beam but the picked off energy is a relatively small fraction (e.g., 5 percent or less) of the energy content of the unsplit beam. Monitor 40 is further described in European patent application no.87310283.4, but it here suffices to state that it continuously observes the characterization of the delivered beam and, via a control and feedback bus 44, communicates with the computer. One or more criteria of beam character and quality in the monitoring sample are evaluated via the computer, and if predetermined tolerance criteria are not met, the computer will not issue a shutter-opening command in control line 37 to the safety shutter 38. Finally, a computer control connection 45 is schematically indicated for concurrent bodily displacement of a beam-sampling splitter 46 into and out of the homogenized circular beam at 10', in coordination with in and out bodily displacement of the mirror 42 with respect to a split sample path 47 to the beam monitor 40, whereby, when desired, the beam at 10' can be verified for its adherence to tolerance limits, as a further precondition of beam characterization and delivery to the eye.

Returning to Fig. 2, beam-homogenizing and shaping means 22 is seen to include a beam-expanding pair of anamorphic elements 50-51, a scraper 53 having an aperture configured to reduce the transmitted laser beam to a circular section (with deflection of the unused remainder of the beam to a trap 54), an optical filter 55, and a beam-rotating device 52. More particularly, the anamorphic beam-expander elements 50-51 may be cylindrical lenses, or prisms (as shown), suitably of right-triangular section, with 45° corner angles. Prisms 50-51 are oriented to expand the H dimension to the extent of converting the rectangular-section beam of Fig. 3a to the square-section beam of Fig. 4a, i.e., wherein the expanded H dimension equals W; the resultant beam-intensity profiles in the X and Y directions are schematically indicated in Figs. 4b and 4c.

Having been scraped at 53 to a circular section, which is preferably substantially tangent to the respective sides of the square section (Fig. 4a), the beam is generally as displayed in Figs. 5a, 5b, 5c, wherein the dimension W" is substantially the dimension W' of Fig. 4a. The beam next passes through optical filter 55, with spatially uniform transmission in the direction parallel to the X axis, and with non-uniform but axially symmetric transmission characteristics in the orthogonal direction (Y), see Fig. 6. The non-uniform transmission profile of Fig. 6 is that required to compensate for the truncated quasi-Gaussian profile depicted in Fig. 5c, and the circular beam 10' which results from filter action at 55 has approximately uniform cross-sectional intensity in all directions, as shown schematically in Figs. 7b and 7c, with a sectional diameter W" which is unchanged from that which is incident on filter 55. The beam 10' will thus be understood to have been substantially homogenized, in terms of its flux-density (intensity) distribution, essentially throughout its full circular section, although the value of the intensity will have been attenuated inter alia by means 55 to a re-

duced magnitude I'.

Although the described techniques effect substantial homogeneity in the beam, the fact remains that certain irregularities can characterize the intensity profile for different angular orientations across the beam section, and such irregularities can vary from pulse to pulse, it being implicit in use of the invention to .recontour the cornea that any such surgical procedure involves multiple pulses of laser energy to progressively ablate tissue to predetermined depth. Of course, if the pulse profiles are sufficiently different from pulse to pulse, the cumulative effect of these differences will be negligible, but experience indicates that they can also cumulatively exhibit an irregularity. To offset the degrading effect of this possibility, the invention provides beam-rotating means 52 which may be a refracting prism, such as a "Dove" or "Delta" prism but which is shown as a so-called "K-prism" using three mirrors mounted for continuous unitary rotation about the local axis of beam propagation. In the course of a given period of surgical exposure at 14, the extent of such beam rotation is preferably at least one revolution, thus causing small residual inequalities of intensity across a given orientation of beam diameter to be superposed sequentially at a different relative orientation for each laser pulse, and thereby causing the cumulative surgically delivered energy to become rotationally symmetrical about the axis of propagation after numerous pulses have been transmitted. In Fig. 2, an edge-drive motor 52' is the means of imparting steady rotation to the beam rotator 52, and suitable sensor means symbolized at 52" will be understood to monitor the extent of such rotation in the course of a given surgical procedure, e.g., to so control the speed and time of operation of motor 52' as to achieve a number of complete revolutions of means 52 upon termination of the surgical procedure. Illustratively, a given surgical procedure for cornea-curvature correction will involve a minimum of 500 pulses at a repetition rate of 10/sec., with beam rotator 52 driven at slightly more or slightly less than one revolution per second, such that the pulses of succeeding revolutions are not precisely additive at the same angular orientation of rotator 52.

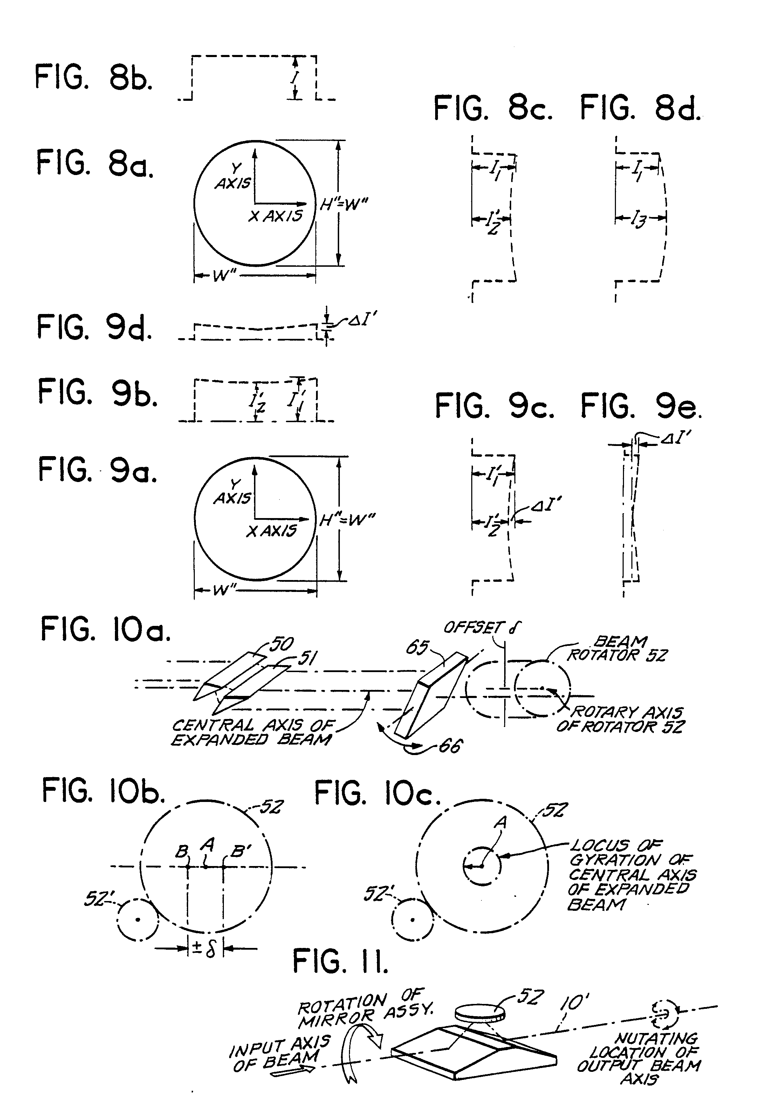

The effect produced by rotator 52 upon the laser beam is depicted in Figs. 8a to 8d for two typical cases of residual non-uniformity, wherein it illustratively appears in one case that the homogeneous situation shown by Figs. 7a, 7b, 7c is marred in the almost-identical situation shown by corresponding Figs. 8a, 8b, 8c by reason of a droop (from $I_1$ to $I_2$) of intensity level in the Y-axis direction (Fig. 8c) although there is no such inequality of intensity distribution in the X-axis direction. But the effect of beam rotation at 52 is seen in Figs. 9a, 9b, 9c to produce identical X and Y intensity profiles, in each of which an $I_1'-I_2'$ irregularity appears, but with much reduced severity $\Delta I'$ compared to the $I_1-I_2$ irregularity of Fig. 8c. Rotational symmetry is thus assured, in spite of the reduced differential $I_1'-I_2'$. But it will be understood that this inequality ($I_1'-I_2'$) can be compensated in the means 23 for specially characterizing the beam profile.

Figs. 9d and 9e show quadrature-component profiles of an attenuating filter having rotational symmetry and configured to attenuate the described effective output of beam rotator 52, to the characterizing extent that the inequality $I_1'-I_2'$, or $\Delta I'$ is effectively neutralized, resulting in a laser-beam section, as described by Figs. 7b, 7c. The attenuating filter of Figs. 9d, 9e may be a separate component, as at 24, or its rotationally symmetrical profile may be a modifying component of the particular curvature-correcting density distribution of each of the different characterizing filters of turret 23.

Another case of irregular intensity distribution (prior to beam rotation) is illustrated by Figs. 8a, 8b, 8d, it being noted that the Y-axis intensity distribution (Fig. 8d) is somewhat domed, to a greater central magnitude $I_3$ than $I_1$ which prevails at upper and lower ends. Again, the effect of processing the beam of Figs. 8a, 8b, 8d through rotator 52 will be to achieve rotational symmetry, the intensity $I_3'$ at the center being less than $I_3$ but equally present in the resulting X and Y intensity profiles (not shown). Again also, the $I_3'$ irregularity, being rotationally symmetrical, is characteristic for a given instrument and is correctable by appropriate design or control of the special characterizing means 23.

It will be understood that the residual intensity inequalities discussed in connection with Figs. 8a, 8b, 8c on the one hand and in connection with Figs. 8a, 8b, 8d on the other hand are merely illustrative, in that the inequalities may be asymmetrical, or offset from or at plural orientations with respect to the local propagation axis. In all cases, the described beam rotation at 52 not only enables enhanced rotation symmetry of the ultimately delivered distribution of surgical energy, but also substantially reduces the degrading effect of such localized variations in intensity as may occur on a pulse-to-pulse basis in the laser beam.

It will be recalled that the scale of the section of beam 10' is large, e.g., 13 or 14-mm diameter, compared with the size, e.g., about 5-mm diameter, which may be prescribed for delivery at 14 to the eye 13. The lower half of Fig. 2 deals with specific illustrative components for specifically characterizing energy distribution across the beam and for reduction of the beam section area for such delivery at 14.

In Fig. 2, the characterizing of energy distribution across the laser-beam section is determined by filter means that is selectively positioned in the beam path, the particular selected filter being as appropriate for the corneal ablation prescribed by the ophthalmologist to correct refractive errors of the patient's eye. This technique is disclosed in greater detail in European patent application no.87306826. Briefly, a selected characterizing filter A is one of a plurality (A to F) carried at equally spaced locations on a disc or turret 56 that is angularly indexable about a fixed axis 57; the diameter of filter openings at locations A to F may desirably be slightly less than that of the beam at 10', e.g., a 0.5-mm reduction in beam diameter, thus providing a tolerance for possible misalignment. The filter openings of turret 56 therefore served to further limit or scrape the beam diameter, when indexed into the axis of beam propagation. Indexing rotation is imparted to disc 56 by edge-drive means 58, in cooperation with the means symbolized at 59 for enabling precise location of each possible index position, it being understood that bus 33 of Fig. 1 places the computer in the control loop which includes the drive and locating means 58-59.

Filter A may be characterized by a transmission profile which passes maximum beam intensity at the center and which progressively attenuates beam intensity as an increasing function of radius about the center; such a filter enables greatest depth of ablation at the center of beam 14, diminishing progressively to zero or substantially zero ablation at the circular periphery of the beam section. Such characterizing filter at A will find utility in spherically sculpturing the cornea to greater radius of curvature, in corrective reduction of myopia.

In analogous manner, characterizing filter at B may be designed to effect spherical corrective reduction of hyperopia, by passing greatest beam intensity at maximum radius of that circular area of the cornea which is to be optically improved; in this case, filter attenuation of the beam increases progressively with decreasing radius, thereby enabling beam 14 to reduce the radius of cornea curvature. In this particular case of hyperopia reduction, it is preferred that the opening at B shall be larger than that at other filter locations, i.e., that opening B shall not be operative to reduce the diameter of the homogenized beam, thus defining an annulus outside the optically corrected area; within this annulus, the attenuation characteristic of filter B preferably increases to maximum at the outer diameter, whereby sharp-edge development can be avoided in the sculptured surface and epithelium regrowth can proceed more rapidly.

In further analogous manner, a characterizing filter at C may be designed to effect a cylindrical curvature correction, as for corrective reduction of an astigmatic condition of eye 13. The filter at C may thus be characterized to transmit the cylindrical laser beam with greatest intensity along a diametric alignment through the center of the filter, and with a laterally symmetrical distribution of progressive beam attenuation which increases with lateral offset from the diametric alignment. The particular orientation of the thus-characterized beam is effected by beam rotator 25, which may be as described for rotator 52 and mounted for rotation about the local axis of the characterized beam; the bus 35 of Fig. 1 will be understood to enable computer control of an edge drive 60, pursuant to angular-position sensing at 61, the same having been selected and set, for a given procedure, in accordance with requirements indicated by prior examination of eye 13.

In preparation of the cornea to receive a transplant, another of the opening locations (e.g., D) may be of diameter which, after reduction at 24, has been predetermined to create a sculpturing removal of corneal tissue to an extent suitable for receiving the transplant.

Remaining turret locations E and F may be equipped with further different filters, e.g., to achieve myopia correction or hyperopia correction via filter-density distributions which are specifically different from those at A and B. Or these positions may be equipped with filter spares, in the event of filter degradation at A or B, in the course of extended usage. In all cases of beam characterizing at the various turret locations A through F, it will be understood any rotationally symmetrical irregularity resulting from operation of the homogenizing rotator 52 may be compensated in the design of the particular turret-indexed filter; alternatively, a single filter to compensate for the rotationally symmetrical irregularity may be a component (not shown) of the beam-condenser means 24.

The description thus far has illustratively assumed that beam profiling for particular different optically correcting laser surgery of the cornea shall employ suitably characterized filters, such as those at the different index locations of turret 23. But the beam-expansion and rotational manipulations described herein for homogenizing the beam, at least to the extent of assuring rotational symmetry of the beam, are also applicable to other techniques of such laser surgery, such as that which is involved in indexed apertures as described in connection with Fig. 2a of European patent application no.87310283.4. In all cases, a single compensating filter of rotational symmetry can be used (as in conjunction with lens means 24) to neutralize irregularity in the rotationally symmetrical effective output of the described rotator 52.

Figs. 10a, 10b, 10c are illustrative of other and/or further means involving beam rotation whereby the laser beam can be effectively homogenized in the course of a given period of corneal-ablation surgery. But before discussing any of these further means, it is well to make certain observations as to effects caused by rotator 52. Rotator 52 will cause the beam to rotate about its axis without linearly translating or angularly tilting if the rotational axis of the rotator assembly coincides accurately with the beam axis and said rotator assembly is properly aligned internally. Internal or external misalignments cause the propagation direction and/or the axis location of the rotated beam 10' to vary as the rotator turns. This behavior is characteristic in all types of rotator means such as the Dove prism, the delta prism or the K-mirror assembly shown as a preferred embodiment in Fig. 2. In the presence of misalignment-induced beam tilt or displacement, the beam axis may be seen to gyrate in the manner described mathematically as the limacon of Pascal, as illustrated schematically in Fig. 11. The locus of the moving axis of beam 10' may then be a single or double circle, or a cardioid, depending upon the magnitudes of the rotator misalignments.

While undesirable in the normal applications of beam rotators in optical instruments, this behavior is advantageous in the present case as a means of intensity-homogenizing the beam presented to the cornea since the cumulative consequences of minor localized beam intensity variations are thereby effectively distributed over an extended region of the cornea. Small misalignments of the rotator are thus a beneficial attribute of the invention.

In the arrangement of Fig. 10a, the scraper 53 and filter 55 of Fig. 2 have been omitted, in illustration of the facts that (a) the effective action of rotator 52 is to produce an output wherein irregularities have rotational symmetry, which we have seen can be compensated at 24 or elsewhere in the optical system, downstream from rotator 52, and (b) circular scraping of the beam can in any event occur at one of the apertures of turret 23. In Fig. 10a, the expanded-beam output of anamorphic elements 50, 51 is transmitted directly to rotator 52, but the central axis of beam output from means 50, 51 is caused to be at offset $\delta$ from the axis of rotation of beam rotator 52. In the form shown, this offset $\delta$, which may be a fixed offset, is produced by interposing a plane-parallel optical prism 65, inclined from normal to the central axis of the expanded beam, between expander means 50, 51 and rotator 52. The effect of rotating the thus-offset beam at 52 will be seen, in the course of rotation, to eccentrically gyrate any intensity irregularity that may be present at the center of the expanded-beam output of means 50, 51, thus effectively dissipating such irregularity in the rotational symmetry which is the integrated product of rotation at 52. A double-headed arrow 66 will be understood to suggest selectively adjustable setting of the magnitude of offset $\delta$.

The diagram of Fig. 10b is taken normal to the axis of beam propagation to and issuing from rotator 52, for the case in which offsets $\delta$ may be effected as a varying function of rotation about the rotary axis A of rotator 52. The limits of such varying offset are identified at B, B', and may be achieved by oscillating tilt of a plate 65 to equal extents on opposite sides of a normal to the central axis of the expanded beam. Preferably, the tilt program for any such oscillation should be such as to avòid synchronism or integer multiple or submultiple relation with the rotational cycle of beam rotator 52.

The diagram of Fig. 10c resembles that of Fig. 10b but indicates modification wherein the offset $\delta$ is itself eccentrically gyrated about the axis of rotation of beam rotator 52, the locus of such gyration being shown by legend in the drawing. The eccentric gyration may be produced by continuous bodily rotation of plate 65 about the central axis of the expanded beam, all the while at fixed tilt from a plane normal to said central axis; the degree of tilt during such bodily rotation may be fixed or it may be variable in the course of a given procedure. Again, it is recommended that the cycle of eccentric gyration be asynchronously related to either the rotary cycle of rotator 52 or to any cycle of varying tilt.

It will be recalled that it is a feature of the invention that the height and width of the expanded beam, as processed by intensity profiling means 23 are substantially greater than the beam-section dimensions at surgical delivery to the eye. In the case of the turret of Fig. 2, each index station provides the particular intensity characterizing function within a circular aperture, the filter action being operative upon laser radiation which is by nature collimated, as indicated by the cylindrical column of rays to and through rotator 25 for incidence upon beam-condenser means 24. Means 24 is seen by convergent ray output beam to bring the laser radiation to a focal point at safe offset from the eye 13, whereby a divergent laser-ray bundle is incident upon the eye. The diameter of the target circle within which laser action is to proceed at the' eye will be understood to be a function of such adjusted positioning of means 24 along the optical axis as will enable means 24 to place a correctly sized image of the involved turret aperture at the eye, all as illustrated by the fragmentary diagram of Fig. 2a, for the assumed case of filter aperture A imaged at the eye 13. With the aperture A thus focused to specified size at the cornea, the characterized laser ablation will be understood to proceed under action by divergent rays which safely avoid bringing the laser beam to focus at the eye.

The safety shutter 38 is schematically shown for fail-safe operation, being a blade 62 pivoted on a fixed axis and continuously biased by a tension spring 63 toward its elevated position 62', in which beam 14 is cut off. It is only when an actuating solenoid 64 is actuated, by a command signal in line 37, that blade 62 can be displaced downward, to the position shown in solid outline, to allow beam 14 delivery to eye 13.

Beam monitoring, alluded to at 40 in Fig. 1, is not a part of the present invention although it is useful in monitoring the fidelity of beam homogeneity and/or special intensity characterization achieved by the described apparatus. Such beam monitoring is described in detail in European Patent application no.87310283.4 and, therefore, need not be here repeated. However, it will be understood that since the action of rotator 52 is to achieve rotational symmetry through the integrated effect of serving multiple pulses for at least one full rotation at 52, it will be necessary to incorporate a storage capability in the monitoring function whereby the "line grabber" and/or "frame-grabber" functions described in European patent application no.87310283.4 become "grabbers" of the averaged (integrated) product of laser exposure throughout at least one full revolution of rotator 52. And if the speed of rotation at 52 is selected to accomplish plural revolutions in the course of a given treatment, then upon completion of a single revolution, there can be a sufficient accumulation of stored data to enable a monitoring display from which to determine whether the particular surgical procedure has begun and is proceeding as it was intended by the surgeon. The surgeon thus has the opportunity to make his decision to abort long before completion of the full surgical treatment, should he decide that the monitored delivery of laser energy distribution is less than satisfactory for the intended purpose.

The described invention will be seen to meet all stated objects and to provide the surgeon with a precision tool for enhanced quality and safety of corneal-sculpture procedures. Optical system components, including for example the anamorphic components 50-51 and the lens components, are available from various suppliers such as Melles Griot, and it is recommended that they be of vacuum ultraviolet-grade fused silica, preferably coated, as appropriate for the involved laser-beam wavelength.

It is to be understood that, for simplifying purposes, the surgical procedures which have been discussed have assumed that optical correction of a given eye involves only elimination or reduction of optical errors attributable to the topography of the cornea, thus avoiding discussion of such further optical errors as may be contributed by the inner natural lens of a given eye. It will be understood, therefore, that the corneal topography data ascertained at 17 is to be taken in context with data from prior examination of the eye's overall performance. From such examination, the extent of required correction is ascertained; corneal-sculpting procedure commences with the measured topography data as a starting point, and must follow such a prescribed combination of spherical and/or cylindrical ablative procedures as will best serve, through sculpted modification of corneal topography, as to tolerably achieve the desired overall correction of the eye's performance.

## Claims

1. In apparatus using an ultraviolet laser to correct an optically deficient eye by volumetric ablative removal of corneal tissue from the anterior surface and with penetration of the stroma, wherein laser-beam delivery is on an optical path which terminates with a fixed cornea-impingement axis aligned with the axis of the eye, and wherein flux distribution is so characterized within a predetermined circle of laser-radiation exposure to the cornea as in the course of a predetermined exposure time to so distribute the cumulative depth of ablation as to achieve a new and improved corneal curvature, the improvement wherein the laser is an excimer laser producing a beam of section which is elongate in the width dimension and which is relatively narrow in the height dimension, and wherein beam-homogenizing means is on said path downstream from the laser and upstream with respect to the characterizing of flux distribution, said beam-homogenizing means comprising anamorphic beam-expansion means oriented to expand the relatively narrow height dimension to substantially the elongate extent of the width dimension, and beam-rotation means positioned to rotate the expanded beam about a generally central axis prior to characterizing the flux distribution within the predetermined circle.

2. The improved apparatus of claim 1, in which the axis of beam rotation is substantially coincident with the central axis of the expanded beam.

3. The improved apparatus of claim 1, in which the axis of beam rotation is offset from the central axis of the expanded beam and/or is tilted with respect thereto.

4. The improved apparatus of claim 3, in which said offset and/or tilt is fixed.

5. The improved apparatus of claim 3, in which said offset and/or tilt is variable in a fixed direction transverse to the axis of the expanded beam.

6. The improved apparatus of claim 1, in which said beam-rotation means is positioned to eccentrically gyrate the expanded beam about an axis which optically intersects the center of the predetermined circle.

7. The improved apparatus of any one of claims 1 to 6, in which said beam-homogenizing means includes a scraper for limiting the homogenized beam to a beam of circular section, whereby flux distribution may be characterized over the circular section.

8. The improved apparatus of any one of claims 1 to 6, in which said beam-homogenizing means includes a scraper for limiting the homogenized beam to a beam of circular section, said scraper being interposed between said beam-expansion means and said beam-rotation means, whereby flux distribution may be characterized over the circular section.

9. The improved apparatus of any one of claims 1 to 6, in which said beam-homogenizing means includes a scraper for limiting the homogenized beam to a beam of circular section, said scraper being at a location downstream from said beam-rotation means, whereby flux distribution may be characterized over the circular section.

10. The improved apparatus of any one of claims 1 to 6, in which said beam-homogenizing means includes a scraper for limiting the homogenized beam to a beam of circular section, said scraper being defined by a circular filter aperture wherein the filter is characterized by an attenuation profile selected for achievement of a predetermined change in cornea curvature.

11. The improved apparatus of any one of claims 1 to 6, in which said beam-homogenizing means includes a filter having an attenuation profile of rotational symmetry selected to effectively compensate a rotationally symmetrical irregularity in the time-integrated sectional intensity profile of laser-beam propagation issuing from said beam-rotation means.

12. The improved apparatus of any one of claims 1 to 6, in which said beam-homogenizing means includes a filter having an attenuation profile of rotational symmetry selected to effectively compensate a rotationally symmetrical irregularity in the time-integrated sectional intensity profile of laser-beam propagation issuing from said beam-rotation means, the characterizing of flux distribution to achieve a new and improved corneal curvature being a filter having a characterizing attenuation profile in which said attenuation profile of rotational symmetry is embodied as a corrective component.

13. The improved apparatus of claim 1, (a) in which the height and width dimensions of the expanded beam are substantially greater than the beam-section dimensions at delivery to the eye, (b) in which beam characterization is at a circular aperture, and (c) in which beam-condenser means reduces said aperture to said predetermined circle and comprises lens means imaging said aperture at or near the eye, whereby the focal point for laser radiation occurs at an axial offset from beam incidence upon the eye.

14. The method of preparing an ultraviolet laser beam for intensity-distributed characterization over a circular section area in order that the characterized circular beam may be used to effect a curvature change in the anterior surface of a cornea, thereby to change optical properties of an eye by selectively ablating the anterior surface with penetration into the stroma to achieve over a period of treatment time a volumetric removal of corneal tissue, which method comprises selecting an excimer laser to produce an output beam of generally rectangular two-dimensional section wherein one of the dimensions substantially exceeds the other dimension, optically expanding said other dimension to substantially the extent of said one dimension, whereby the beam section has quadrature profiles of substantially equal extent on opposite sides of the central axis of an expanded beam, and optically rotating the expanded beam, thereby in the course of such optical rotation to effectively deliver for subsequent intensity-distributed characterization a beam having rotational symmetry.

15. The method of claim 14, including the further step of filtering the beam which results from beam rotation, the filtering step being according to an attenuation profile of rotational symmetry, which profile is selected to compensate for an irregularity of circular symmetry resulting from beam rotation.

16. Laser apparatus for ophthalmological surgery including beam rotation means provided in the laser beam path for alleviating the effects of irregular beam intensity distribution across the beam cross-section.

FIG. 1.

FIG. 2.

FIG. 2a.

FIG. 3b.

FIG. 3a.

FIG. 4b.

FIG. 4a.

FIG. 3c.

FIG. 4c.

FIG. 5b.

FIG. 5a.

FIG. 7b.

FIG. 7a.

FIG. 5c.

FIG. 6.

FIG. 7c.

0 280 414

**FIG. 8b.**

**FIG. 8c.**   **FIG. 8d.**

**FIG. 8a.**

Y AXIS
X AXIS
$H''=W''$
$W''$

**FIG. 9d.**   $\Delta I'$

**FIG. 9b.**   $I'_2$   $I'_1$

**FIG. 9c.**   **FIG. 9e.**
$\Delta I'$

**FIG. 9a.**

Y AXIS
X AXIS
$H''=W''$
$W''$

$I'_1$
$I'_2$   $\Delta I'$

**FIG. 10a.**

50
51
65   OFFSET $d$   BEAM ROTATOR 52
CENTRAL AXIS OF EXPANDED BEAM
66
ROTARY AXIS OF ROTATOR 52

**FIG. 10b.**   **FIG. 10c.**

52
B A B'
52'
$\pm\delta$

52
A
LOCUS OF GYRATION OF CENTRAL AXIS OF EXPANDED BEAM
52'

**FIG. 11.**

ROTATION OF MIRROR ASSY.
52   10'
INPUT AXIS OF BEAM
NUTATING LOCATION OF OUTPUT BEAM AXIS

# DOCUMENTS CONSIDERED TO BE RELEVANT

| Category | Citation of document with indication, where appropriate, of relevant passages | Relevant to claim | CLASSIFICATION OF THE APPLICATION (Int. Cl. 4) |
|---|---|---|---|
| Y,P | EP-A-0 224 322 (P.A. CONSULTING SERVICES LTD)<br>* Abstract; figures 1,4-9,14,16-19 *<br>--- | 1-15 | A 61 F 9/00<br>G 02 B 27/00 |
| Y | WO-A-8 700 038 (R. THYZEL)<br>* Whole document *<br>--- | 1-15 | |
| A | EP-A-0 019 778 (IBM CORP.)<br>* Abstract *<br>--- | 1 | |
| A | EP-A-0 171 888 (MELLES GRIOT, IRVINE CO.)<br>* Figures 5,6,11,12 *<br>----- | 1 | |

|  |  |  | TECHNICAL FIELDS SEARCHED (Int. Cl.4) |
|---|---|---|---|
|  |  |  | A 61 F<br>G 02 B<br>H 01 S |

The present search report has been drawn up for all claims

| Place of search | Date of completion of the search | Examiner |
|---|---|---|
| THE HAGUE | 15-05-1988 | ARGENTINI A. |

EPO FORM 1503 03.82 (P0401)